# EUROPEAN PATENT SPECIFICATION

(11) **EP 3 922 298 B1**
(45) Date of publication and mention of the grant of the patent: **28.06.2023**
(21) Application number: 20760265.7
(22) Date of filing: 06.02.2020
(51) Int. Cl.: A61M 39/28, A61M 1/02

(54) **BLOOD BAG SYSTEM AND CLAMP**
BLUTBEUTELSYSTEM UND KLEMME
SYSTÈME DE SAC DE SANG ET AGRAFE

(30) Priority: 20.02.2019 JP 2019027946
(43) Date of publication of application: 15.12.2021
(73) Proprietor: TERUMO Kabushiki Kaisha, Tokyo 151-0072 (JP)
(72) Inventor: NAKANO, Masanori, Fujinomiya-shi, Shizuoka 418-0004 (JP)
(74) Representative: TBK
(86) International application number: PCT/JP2020/004484
(87) International publication number: WO 2020/170832

(56) References cited:
- WO-A1-2015/129520
- WO-A1-2015/129520
- WO-A1-2018/088072
- WO-A1-2018/088072
- JP-A- 2007 512 106
- US-A- 3 316 935
- US-A- 3 316 935
- US-A1- 2017 043 111
- US-A1- 2017 043 111
- US-B1- 6 840 492

## Description

### Technical Field

The present invention relates to a blood bag system and a clamp.

### Background Art

For example, JP 2016-106012 A discloses a blood bag system including a first bag (parent bag) for containing blood, a second bag (child bag) for containing a blood component obtained by centrifuging the blood in the first bag, and a third bag (liquid medicine bag) for containing an additive solution.

A first tube connected to the first bag is connected to a second tube connected to the second bag and a third tube connected to the third bag via a branching section.
Document US 3 316 935 A (D1) discloses a sliding clamp having three narrowing slits directed to different directions. The clamp is inserted into and passing through a slit of a body member having three holes for accommodating respective tubes. Two of the slits are directed to one direction, while the other slit is directed to the opposite direction. Correspondingly, it is not possible to close each of three tubes inserted through one of the slits each at the same time. Moreover, a certain operating section appears not present.
Document WO 2015/129520 A1 (D2) pertains to a blood bag system.
Document WO 2018/088072 (D3) in a similar manner to that of US 3 316 935 A discloses a sliding clamp having a configuration where two slits are provided. Both of these slits narrow to both of their ends. A widened center portion in each of the slits is configured to accommodate two tubes adjacent to each other. Thus, a total of four tubes can be accommodated in the sliding clamp. Upon activating of the clamp, one of the two tubes arranged in a slit becomes closed while the second tube is maintained in an open state. Upon shifting the clamp to the opposite side, the closed tube becomes opened while the opened tube becomes closed.
Document US 6 840 492 B1 (D4) discloses a sliding clamp having one slit which is provided with a narrowing section. At the boundary portion from the slit to the narrowing section an elastic retaining portion is provided. Thus, shifting of the tube including undesired opening is prevented, but upon application of a slightly higher shifting force, the tube can be opened again.

### Summary of Invention

In the above-described blood bag system, the blood bag system is set in a centrifuge/delivery apparatus to centrifuge the blood in the first bag. Then, a blood component obtained by centrifugation is delivered from the first bag to the second bag via the first tube and the second tube. Thereafter, the blood bag system is removed from the centrifuge/delivery apparatus in a state where a flow channel of the second tube is closed by a first clamp and a flow channel of the third tube is closed by a second clamp. Subsequently, the third bag is suspended from a suspension stand, the second clamp is removed from the third tube, and the additive solution, such as a red blood cell preservation solution, is delivered to the first bag via the third tube and the first tube.

In this manner, when the first clamp for closing the flow channel of the second tube and the second clamp for closing the flow channel of the third tube are separate parts, there is a possibility that it is difficult to efficiently close the respective flow channels of the two tubes (the second tube and the third tube).

The present invention has been made in view of such problems, and has the object to provide a blood bag system and a clamp capable of efficiently closing the respective flow channels of two tubes by one clamp.
The object of the invention is achieved by a blood bag system according to claim 1. Advantageous embodiments are carried out according to the dependent claims.

An aspect of the present invention is a blood bag system including: a first bag for containing blood; a second bag for containing a blood component obtained by centrifuging the blood in the first bag; a third bag for containing an additive solution; and one clamp capable of closing respective flow channels of a second tube and a third tube. A first tube connected to the first bag is connected to the second tube connected to the second bag and the third tube connected to the third bag via a branching section. The clamp includes a base formed with an insertion hole through which the second tube and the third tube are inserted. A wall section forming the insertion hole is provided with: a first slit for pressing an outer circumferential surface of the second tube inward so as to close the flow channel of the second tube; and a second slit for pressing an outer circumferential surface of the third tube inward so as to close the flow channel of the third tube.

According to the present invention, the second tube and the third tube are inserted into the insertion hole formed in the base, and the first slit and the second slit are provided in the wall section forming the insertion hole. Thus, the respective flow channels of the second tube and the third tube can be efficiently closed by the single clamp.

### Brief Description of Drawings

Fig. 1 is an explanatory view illustrating an overall configuration of a blood bag system and a centrifuge/delivery apparatus according to an embodiment of the present invention.
Fig. 2 is a plan view illustrating a unit set area of the centrifuge/delivery apparatus.
Fig. 3A is a perspective explanatory view of a clamp of Fig. 1, and Fig. 3B is a plan explanatory view of the clamp of Fig. 1.
Fig. 4 is an explanatory view of a procedure for delivering a red blood cell preservation solution from a liquid medicine bag to a blood bag.
Fig. 5A is an explanatory plan view of a first slit according to a first configuration example, Fig. 5B is an explanatory plan view of a first slit according to a second configuration example, and Fig. 5C is an explanatory plan view of a first slit according to a third configuration example.
Fig. 6A is an explanatory plan view of a first slit according to a fourth configuration example, and Fig. 6B is an explanatory plan view of a first slit according to a fifth configuration example.
Fig. 7 is an explanatory plan view of a clamp according to a first modification.
Fig. 8A is a perspective explanatory view of a clamp according to a second modification, Fig. 8B is a plan explanatory view of the clamp of Fig. 8A, and Fig. 8C is an explanatory view of a configuration example of an operation section forming the clamp of Fig. 8A.
Fig. 9 is an explanatory plan view of a clamp according to a third modification.

### Description of Embodiments

Hereinafter, preferred embodiments of the present invention will be described in detail with reference to the accompanying drawings.

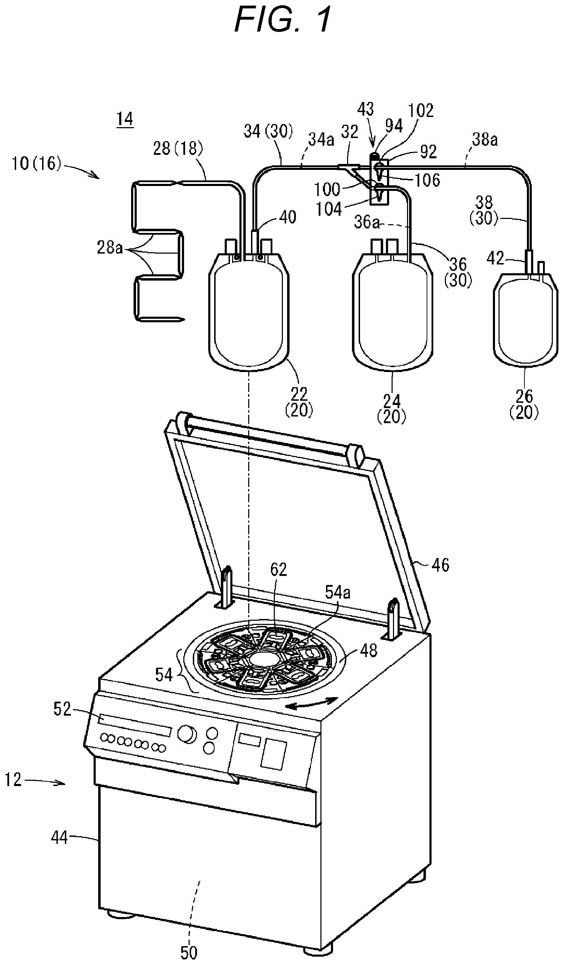

A blood bag system 10 according to an embodiment of the present invention is set in a centrifuge/delivery apparatus 12 as illustrated in Fig. 1 by a user such as a medical worker. The centrifuge/delivery apparatus 12 centrifuges blood of the blood bag system 10 to generate and store a plurality of types of blood components. Hereinafter, a configuration including the blood bag system 10 and the centrifuge/delivery apparatus 12 is referred to as a blood product manufacturing system 14.

The blood bag system 10 includes a pretreatment unit (not illustrated) used for blood sampling before centrifugation and a separator unit 16 that generates blood components by centrifugation and individually stores the blood components, and the both units are connected by a relay tube 18. The separator unit 16 is set in the centrifuge/delivery apparatus 12 in a state where the relay tube 18 connected to the pretreatment unit is cut into the state illustrated in Fig. 1 before the centrifugation.

The pretreatment unit of the blood bag system 10 collects whole blood from a donor (blood donor) and removes a predetermined blood component (for example, a white blood cell) from the whole blood. Therefore, the pretreatment unit includes a blood collection needle, a blood collection bag, an initial flow blood bag, a filter (for example, a white blood cell removal filter), and the like, and is configured by connecting the respective members via a plurality of tubes. The separator unit 16 is connected to the downstream side of the filter via the relay tube 18. Specifically, the pretreatment unit stores the first blood out of the whole blood of the donor, collected through the blood collection needle, in the initial flow blood bag, and then stores the remaining blood in the blood collection bag. Further, the pretreatment unit removes white blood cells in the filter by causing the whole blood, stored in the blood collection bag, to flow to the filter, and delivers the removed blood (white-blood-cell-removed blood) to the separator unit 16.

On the other hand, the separator unit 16 of the blood bag system 10 includes a plurality of bags 20 (a blood bag 22, a PPP bag 24, and a liquid medicine bag 26), and is configured by connecting the respective bags 20 via a plurality of tubes 30.

The blood bag 22 (first bag) is directly connected to the relay tube 18 connected to the pretreatment unit in a state of the blood bag system 10 provided as a product. Therefore, the blood bag 22 stores the removed blood delivered from the filter during blood sampling. The blood bag 22 is set in the centrifuge/delivery apparatus 12, and a centrifugal force is applied by the operation of the centrifuge/delivery apparatus 12. As a result, the removed blood in the blood bag 22 is centrifuged into blood components (platelet poor plasma (PPP), red blood cells (RBC)) and the like having different specific gravities. Then, the blood bag 22 delivers PPP under the operation of the centrifuge/delivery apparatus 12 after centrifugation to store only the remaining RBC.

The PPP bag 24 (second bag) stores PPP by receiving the PPP supplied from the blood bag 22. On the other hand, the liquid medicine bag 26 (third bag) contains a red blood cell preservation solution (additive solution), such as a MAP solution, an SAGM solution, and OPTISOL, in advance.

In addition, a segment tube 28 is formed before the separator unit 16 is set in the centrifuge/delivery apparatus 12. The segment tube 28 has a plurality of sealed tubes 28a in which blood of the donor (removed blood) exists. The plurality of sealed tubes 28a are formed so as to be continuous in series by cutting the relay tube 18, which connects the blood bag 22 and the pretreatment unit (filter), near the filter and further sealing the cut tube at predetermined intervals. The segment tube 28 is cut by a user as necessary, and is used for checking blood or the like.

The plurality of tubes 30 of the separator unit 16 branch into a plurality of tubes via a branching connector 32 (Y-type connector) as a branching section. Specifically, the plurality of tubes 30 include a first tube 34 connecting the blood bag 22 and the branching connector 32, a second tube 36 connecting the PPP bag 24 and the branching connector 32, and a third tube 38 connecting the liquid medicine bag 26 and the branching connector 32. The first tube 34 has a first flow channel 34a, the second tube 36 has a second flow channel 36a, and the third tube 38 has a third flow channel 38a. Each of the first tube 34, the second tube 36, and the third tube 38 has flexibility. The first flow channel 34a, the second flow channel 36a, and the third flow channel 38a communicate with each other via a flow channel in the branching connector 32.

A breakable sealing member 40 (click tip) is provided at an end of the first tube 34 on the blood bag 22 side. Similarly, a breakable sealing member 42 is provided at an end of the third tube 38 on the liquid medicine bag 26 side. The sealing members 40 and 42 block the first flow channel 34a and the third flow channel 38a until a breaking operation is performed, and prevent a liquid in the blood bag 22 and the liquid medicine bag 26 from being delivered to the other bag 20.

The separator unit 16 includes a clamp 43 that closes at least the respective flow channels (the second flow channel 36a and the third flow channel 38a) of the second tube 36 and the third tube 38. A configuration of the clamp 43 will be described later.

On the other hand, the centrifuge/delivery apparatus 12 in which the separator unit 16 of the blood bag system 10 is set includes a box-shaped base body 44, a lid 46 capable of opening and closing an upper surface of the base body 44, and a centrifugal drum 48 provided on the base body 44. In addition, the base body 44 of the centrifuge/delivery apparatus 12 is provided with a motor (not illustrated) that rotates the centrifugal drum 48, a control unit 50 that controls the operation of the centrifuge/delivery apparatus 12, and an operation display unit 52 configured to allow the user to perform confirmation and operation.

The centrifugal drum 48 has a plurality of (six) unit set areas 54 in which the separator unit 16 can be set. A height of one unit set area 54 is longer than a longitudinal length of the bag 20, and is set in a range of 60° with respect to a rotation center of the centrifugal drum 48. That is, the six unit set areas 54 are arrayed without a gap along the circumferential direction to form the centrifugal drum 48 which is an annular structure.

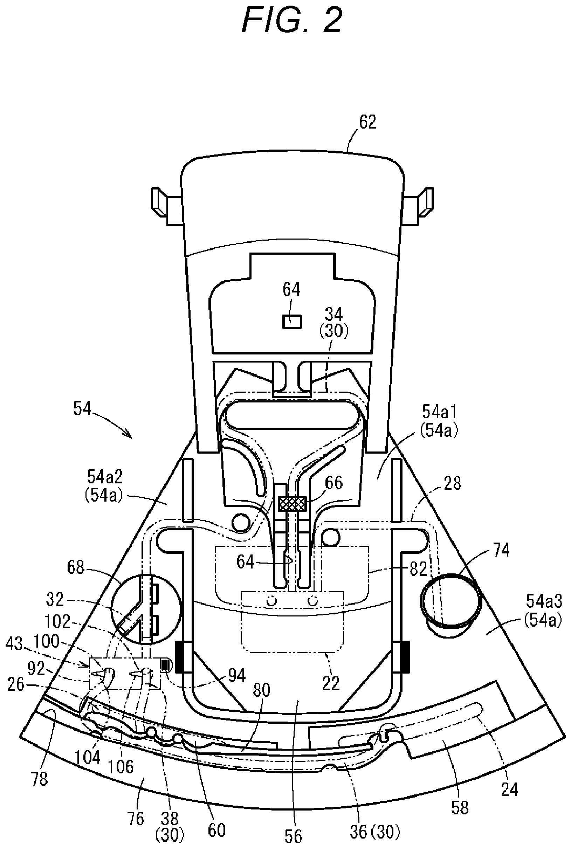

As illustrated in Figs. 1 and 2, the unit set area 54 applies the centrifugal force to the blood bag 22 as the centrifugal drum 48 rotates. Specifically, the unit set area 54 includes a blood bag pocket 56 accommodating the blood bag 22, a PPP bag pocket 58 accommodating a PPP bag 24, and a liquid medicine bag pocket 60 accommodating the liquid medicine bag 26 at radially outer positions of the centrifugal drum 48.

The blood bag pocket 56 is provided at the circumferential center of the unit set area 54 and has a larger volume than the PPP bag pocket 58 or the liquid medicine bag pocket 60. The PPP bag pocket 58 and the liquid medicine bag pocket 60 are provided side by side in the circumferential direction on the radially outer side of the blood bag pocket 56.

In addition, an upper surface 54a of the unit set area 54 is configured such that the plurality of tubes 30 of the blood bag system 10 are arranged and held thereon. The first tube 34 and a part of the segment tube 28 are arranged in a central region 54a1 (first region) on the radially inner side of the blood bag pocket 56 on the upper surface 54a. The central region 54a1 is provided with a lid body 62 that opens and closes an opening of the blood bag pocket 56. Further, a sensor 66 (optical sensor), which detects a part of a breaking section 64 that breaks the sealing member 40 and a state of blood flowing through the first tube 34, is provided at an arrangement position of the first tube 34 closed by the lid body 62.

A part of the first tube 34 passing through the central region 54a1, the branching connector 32, a part of the second tube 36, and a part of the third tube 38 are arranged in a left region 54a2 (second region) of the upper surface 54a. In the left region 54a2, a holder 68 that holds the branching connector 32 is provided.

A part of the segment tube 28 passing through the central region 54a1 is arranged in a right region 54a3 (third region) of the upper surface 54a. In the right region 54a3, a segment pocket 74 that accommodates the plurality of sealed tubes 28a of the segment tube 28 is provided.

Further, a tube holding section 76 protruding upward from the upper surface 54a is provided on the radially outer side of the PPP bag pocket 58 and the liquid medicine bag pocket 60 of the unit set area 54. The tube holding section 76 has an outer wall 78 continuous with an outer peripheral edge of the unit set area 54 and an inner wall 80 protruding from the outer peripheral edge to the inner side of the outer wall 78, and the second tube 36 is arranged between the outer wall 78 and the inner wall 80. The inner wall 80 extends from the left side of the liquid medicine bag pocket 60 to a circumferential intermediate position of the PPP bag pocket 58, and guides the second tube 36 to the PPP bag pocket 58.

In addition, the unit set area 54 includes a slider 82 (pusher), which presses the blood bag 22 after centrifugation, on the radially inner side of the blood bag pocket 56. The slider 82 moves forward and backward along the radial direction of the centrifugal drum 48 under the control of the control unit 50 (see Fig. 1).

In the above-described unit set area 54, the blood bag 22 storing the removed blood, the empty PPP bag 24, and the liquid medicine bag 26 storing the red blood cell preservation solution are accommodated in the blood bag pocket 56, the PPP bag pocket 58, and the liquid medicine bag pocket 60, respectively, by the user. Then, the unit set area 54 centrifuges the removed blood in the blood bag 22 by the rotation of the centrifugal drum 48 and advances the slider 82 after the centrifugation to press the blood bag 22. As a result, the PPP generated by the centrifugation in the blood bag 22 is delivered to the PPP bag 24, and RBC remains in the blood bag 22 after delivering the PPP. Thereafter, the blood bag system 10 is taken out from the centrifuge/delivery apparatus 12 by the user, and the liquid medicine bag 26 is suspended from a stand (not illustrated). As a result, the red blood cell preservation solution is supplied from the liquid medicine bag 26 to the blood bag 22, and the blood bag 22 is turned into a state of storing the RBC (blood products) containing a liquid medicine.

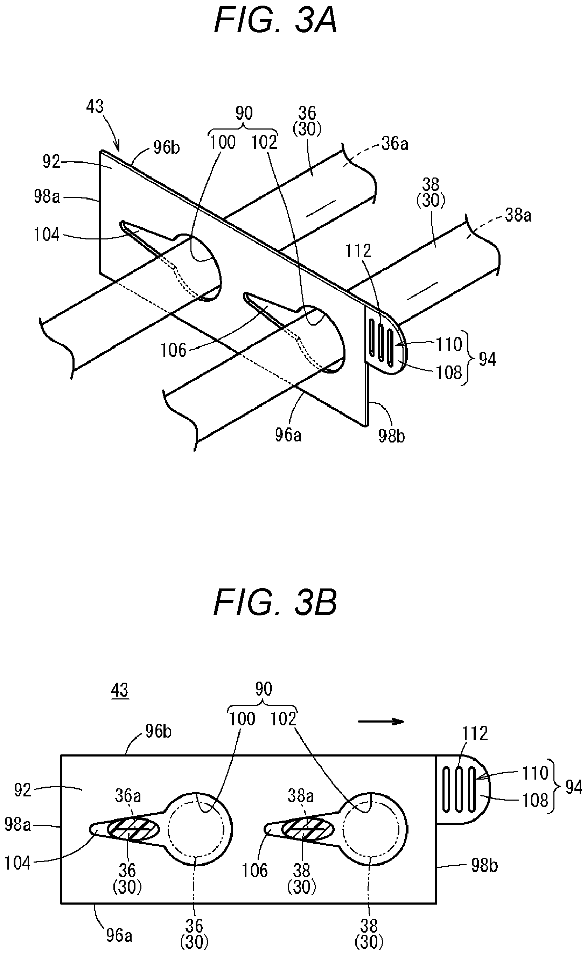

As illustrated in Figs. 3A and 3B, the clamp 43 of the blood bag system 10 includes: a base 92 formed with insertion holes 90 through which the second tube 36 and the third tube 38 are inserted; and an operation section 94 which is provided on the base 92 and can be operated by a finger. That is, the clamp 43 is provided on the second tube 36 and the third tube 38. The base 92 is formed in a rectangular plate shape using a hard resin material. That is, the base 92 includes a first long side 96a and a second long side 96b extending in parallel with each other, and a first short side 98a and a second short side 98b extending in parallel with each other.

The insertion holes 90 include a first insertion hole 100 through which the second tube 36 is inserted and a second insertion hole 102 through which the third tube 38 is inserted. A diameter of the first insertion hole 100 is formed to be equal to or larger than an outer diameter of the second tube 36. A diameter of the second insertion hole 102 is formed to be equal to or larger than an outer diameter of the third tube 38. The first insertion hole 100 and the second insertion hole 102 are arranged along the longitudinal direction of the base 92. The first insertion hole 100 and the second insertion hole 102 are separated from each other by a wall section of the base 92.

A wall section forming the first insertion hole 100 is provided with a first slit 104 for pressing an outer circumferential surface of the second tube 36 inward such that the second flow channel 36a of the second tube 36 is closed. The first slit 104 extends from the first insertion hole 100 toward the side opposite to the second insertion hole 102 (first short side 98a). The first slit 104 linearly extends along the longitudinal direction of the base 92 (a direction in which the first insertion hole 100 and the second insertion hole 102 are arrayed).

The first slit 104 is formed to be narrower in the extending direction. A width dimension of an end of the first slit 104 on the first insertion hole 100 side (the maximum width dimension of the first slit 104) is smaller than the outer diameter of the second tube 36. An extension length of the first slit 104 is equal to or longer than the outer diameter of the second tube 36.

A wall section forming the second insertion hole 102 is provided with a second slit 106 for pressing an outer circumferential surface of the third tube 38 inward such that the third flow channel 38a of the third tube 38 is closed. The second slit 106 extends from the second insertion hole 102 toward the first insertion hole 100. The second slit 106 linearly extends along the longitudinal direction of the base 92 (the direction in which the first insertion hole 100 and the second insertion hole 102 are arrayed). The extending direction of the second slit 106 from the second insertion hole 102 is substantially the same as the extending direction of the first slit 104 from the first insertion hole 100.

The second slit 106 is formed to be narrower in the extending direction. A width dimension of an end of the second slit 106 on the second insertion hole 102 side (the maximum width dimension of the second slit 106) is smaller than the outer diameter of the third tube 38. An extension length of the second slit 106 is equal to or longer than the outer diameter of the third tube 38. The second slit 106 is formed such that the third tube 38 can return from the second slit 106 to the second insertion hole 102. That is, the second slit 106 is formed such that the third flow channel 38a of the third tube 38 can be opened as the third tube 38 returns from the second slit 106 to the second insertion hole 102.

The operation section 94 is provided at an end (the second short side 98b) of the base 92 in a direction opposite to the extending direction of the first slit 104 and the second slit 106. Specifically, the operation section 94 is provided at an end of the second short side 98b where the second long side 96b is located. In other words, the operation section 94 is located closer to the second long side 92b than the center of the base 96 in the lateral direction.

The operation section 94 includes a flat plate portion 108 and an anti-slip portion 110 provided on at least one plane of the flat plate portion 108. The flat plate portion 108 extends from the second short side 92b of the base 98 to a side opposite to the first insertion hole 100 and the second insertion hole 102. The anti-slip portion 110 has a plurality of protrusions 112 arranged along the longitudinal direction of the base 92. Each of the protrusions 112 extends along the lateral direction of the base 92.

The anti-slip portion 110 is not limited to the example having the plurality of protrusions 112, and may have any configuration as long as slipping of the finger with respect to the operation section 94 can be suppressed.

The clamp 43 is provided on the second tube 36 and the third tube 38 such that the second long side 96b (operation section 94) is located above the first long side 96a in a state where the blood bag system 10 is set in the centrifuge/delivery apparatus 12.

The blood bag system 10 according to the present embodiment is basically configured as described above, and operations thereof will be described hereinafter.

As described above, the blood bag system 10 stores the removed blood obtained by removing the predetermined component (white blood cell) from the whole blood of the donor in the blood bag 22, as the user (medical worker) uses the pretreatment unit during blood sampling. The blood bag 22 stores blood of an appropriate blood volume (for example, 400 cc) depending on the donor.

Thereafter, the user separates the separator unit 16 of the blood bag system 10 from the pretreatment unit and sets the separator unit 16 in the centrifuge/delivery apparatus 12 in order to centrifuge the removed blood. The blood bag 22 is inserted into the blood bag pocket 56 of the unit set area 54. The first tube 34 is arranged in the central region 54a1 of the upper surface 54a. The liquid medicine bag 26 is inserted into the liquid medicine bag pocket 60, and the PPP bag 24 is inserted into the PPP bag pocket 58. In addition, the branching connector 32 is set in the holder 68, and the clamp 43 is arranged in the vicinity of the holder 68 in the left region 54a2 of the upper surface 54a. Then, when the user closes the lid body 62, the breaking section 64 reliably breaks the sealing member 40 to open the first flow channel 34a of the first tube 34.

After setting the blood bag system 10, the centrifuge/delivery apparatus 12 rotates the centrifugal drum 48 under the control of the control unit 50 to centrifuge the removed blood in the blood bag 22 into the blood components having different specific gravities (PPP, RBC, and the like). After such centrifugation, the centrifuge/delivery apparatus 12 presses the blood bag 22 by the slider 82. As a result, PPP having a light specific gravity flows out from the blood bag 22, and the PPP flows through the first tube 34, the branching connector 32, and the second tube 36 in this order and flows into the PPP bag 24.

When the PPP is delivered from the blood bag 22 to the PPP bag 24, the centrifuge/delivery apparatus 12 retracts the slider 82 such that the blood bag 22 can be taken out from the blood bag pocket 56. Then, the user closes each of the second flow channel 36a of the second tube 36 and the third flow channel 38a of the third tube 38 by the clamp 43.

That is, the user holds the operation section 94 with a finger and pulls the operation section 94 such that the base 92 moves toward the operation section 94 with respect to the second tube 36 and the third tube 38. Then, the second tube 36 inserted through the first insertion hole 100 is inserted into the first slit 104, and the third tube 38 inserted through the second insertion hole 102 is inserted into the second slit 106, as illustrated in Fig. 3B.

When the second tube 36 is inserted into the first slit 104, the second tube 36 is pressed radially inward by the wall section forming the first slit 104 to be compressed and deformed into a flat shape, and thus, the second flow channel 36a of the second tube 36 is closed. When the third tube 38 is inserted into the second slit 106, the third tube 38 is pressed radially inward by the wall section forming the second slit 106 to be compressed and deformed into a flat shape, and thus, the third flow channel 38a of the third tube 38 is closed.

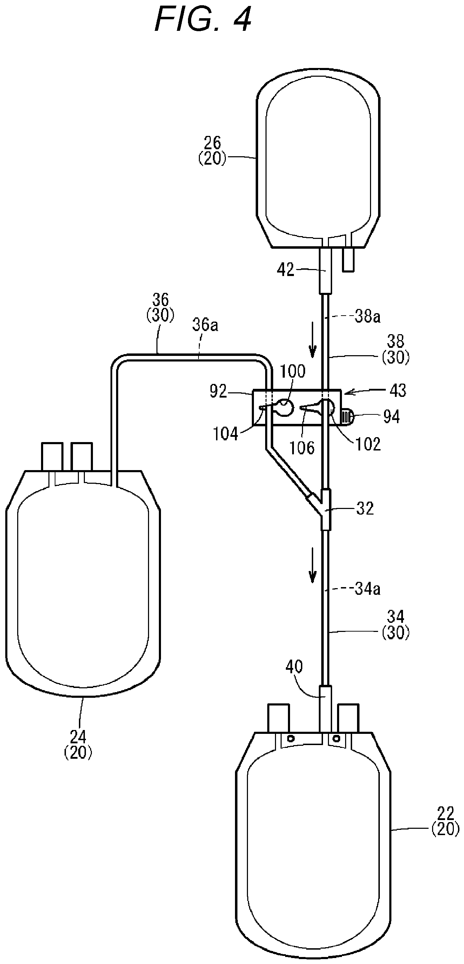

Thereafter, the user takes out the blood bag system 10 from the centrifuge/delivery apparatus 12, and suspends the liquid medicine bag 26 on the stand (not illustrated) as illustrated in Fig. 4. Then, the third tube 38 is moved from the second slit 106 to the second insertion hole 102. That is, the third flow channel 38a of the third tube 38 is opened. As a result, the red blood cell preservation solution in the liquid medicine bag 26 is supplied to the blood bag 22 through the third tube 38, the branching connector 32, and the first tube 34 by the action of gravity. At this time, the second tube 36 remains located in the first slit 104. That is, the second flow channel 36a of the second tube 36 is maintained in the closed state. Therefore, the red blood cell preservation solution is prevented from flowing into the PPP bag 24. As a result, the RBC containing the red blood cell preservation solution is stored in the blood bag 22.

In this case, the blood bag system 10 and the clamp 43 according to the present embodiment have the following effects.

The blood bag system 10 includes: the first bag (blood bag 22) for containing blood, the second bag (PPP bag 24) for containing a blood component obtained by centrifuging the blood in the first bag (blood bag 22), and the third bag (liquid medicine bag 26) for containing the additive solution. The first tube 34 connected to the first bag (blood bag 22) is connected to the second tube 36 connected to the second bag (PPP bag 24) and the third tube 38 connected to the third bag (liquid medicine bag 26) via the branching section (branching connector 32).

The blood bag system 10 includes the single clamp 43 capable of closing the respective flow channels (the second flow channel 36a and the third flow channel 38a) of the second tube 36 and the third tube 38. The clamp 43 includes the base 92 formed with the insertion hole 90 through which the second tube 36 and the third tube 38 are inserted. The wall section forming the insertion hole 90 is provided with the first slit 104 for pressing the outer circumferential surface of the second tube 36 inward such that the flow channel (second flow channel 36a) of the second tube 36 is closed, and the second slit 106 for pressing the outer circumferential surface of the third tube 38 inward such that the flow channel (third flow channel 38a) of the third tube 38 is closed.

According to such a configuration, the second tube 36 and the third tube 38 are inserted into the insertion hole 90 formed in the base 92, and the first slit 104 and the second slit 106 are provided in the wall section forming the insertion hole 90. Thus, the respective flow channels (the second flow channel 36a and the third flow channel 38a) of the second tube 36 and the third tube 38 can be efficiently closed by the single clamp 43.

The second slit 106 is formed such that the flow channel (third flow channel 38a) of the third tube 38 can be opened as the third tube 38 returns from the second slit 106 to the insertion hole 90.

According to such a configuration, the flow channel (third flow channel 38a) of the third tube 38 can be easily closed and opened.

The insertion holes 90 include the first insertion hole 100 through which the second tube 36 is inserted and the second insertion hole 102 through which the third tube 38 is inserted. The first slit 104 is provided in the wall section forming the first insertion hole 100, and the second slit 106 is provided in the wall section forming the second insertion hole 102.

According to such a configuration, the second tube 36 and the third tube 38 can be easily inserted into the first slit 104 and the second slit 106, respectively.

The first slit 104 and the second slit 106 are formed to cause the base 92 to slide in one direction with respect to the second tube 36 and the third tube 38 such that the second tube 36 is inserted into the first slit 104 and the third tube 38 is inserted into the second slit 106.

According to such a configuration, the respective flow channels (the second flow channel 36a and the third flow channel 38a) of the second tube 36 and the third tube 38 can be efficiently closed.

The extending direction of the first slit 104 from the first insertion hole 100 is substantially the same as the extending direction of the second slit 106 from the second insertion hole 102.

According to such a configuration, the second tube 36 and the third tube 38 can be easily inserted into the first slit 104 and the second slit 106, respectively, by causing the base 92 to slide in one direction with respect to the second tube 36 and the third tube 38.

Each of the first slit 104 and the second slit 106 extends along the direction in which the first insertion hole 100 and the second insertion hole 102 are arrayed.

According to such a configuration, the second tube 36 and the third tube 38 can be inserted into the first slit 104 and the second slit 106, respectively, by causing the base 92 to slide along the array direction of the first insertion hole 100 and the second insertion hole 102.

The clamp 43 includes the operation section 94 which is provided at the end of the base 92 in a direction opposite to the extending direction of the first slit 104 and the second slit 106 and can be operated by a finger.

According to such a configuration, the sliding operation of the base 92 can be easily performed.

The operation section 94 includes the flat plate portion 108.

According to such a configuration, the sliding operation of the base 92 can be easily performed.

In the blood bag system 10, once the second flow channel 36a of the second tube 36 is closed by the clamp 43 after delivering the PPP in the blood bag 22 to the PPP bag 24, the second flow channel 36a of the second tube 36 is not opened in a subsequent process. Therefore, the clamp 43 may include first slits 104a to 104e according to first to fifth configuration examples, which will be described hereinafter, for preventing the second tube 36 from returning from the first slit 104 to the first insertion hole 100, instead of the first slit 104.

### (First Configuration Example)

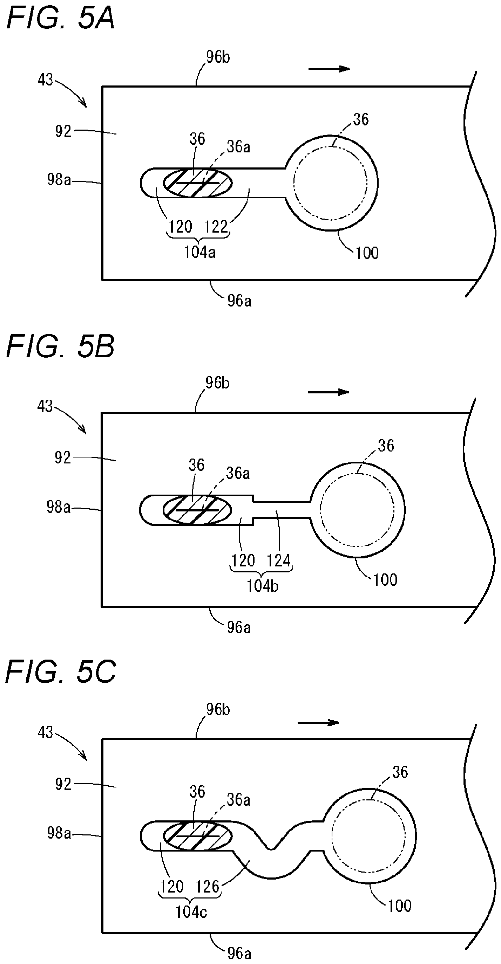

As illustrated in Fig. 5A, the first slit 104a according to the first configuration example includes a holding slit 120 that holds the second tube 36 and an intermediate slit 122 located between the holding slit 120 and the first insertion hole 100. The intermediate slit 122 linearly extends from the first insertion hole 100 toward the first short side 98a. The holding slit 120 linearly extends from an extending end of the intermediate slit 122 toward the first short side 98a. A slit width of the intermediate slit 122 is set to be the same as a slit width of the holding slit 120.

In this case, the first slit 104a includes the holding slit 120 that holds the second tube 36 and the intermediate slit 122 located between the holding slit 120 and the first insertion hole 100, and the slit width of the intermediate slit 122 is substantially the same as the slit width of the holding slit 120. Therefore, the flow channel (second flow channel 36a) of the second tube 36 can be maintained in a closed state with a simple configuration.

### (Second Configuration Example)

As illustrated in Fig. 5B, the first slit 104b according to the second configuration example includes an intermediate slit 124, instead of the intermediate slit 122 of the first slit 104a according to the first configuration example described above. A slit width of the intermediate slit 124 is narrower than the slit width of the holding slit 120.

In this case, the slit width of the intermediate slit 124 is smaller than the slit width of the holding slit 120. Therefore, it is possible to effectively prevent the second tube 36 from returning from the holding slit 120 to the first insertion hole 100.

### (Third Configuration Example)

As illustrated in Fig. 5C, the first slit 104c according to the third configuration example includes an intermediate slit 126, instead of the intermediate slit 122 of the first slit 104a according to the first configuration example described above. The intermediate slit 126 extends to be bent (curved). In other words, the intermediate slit 126 extends in a curve shape (arc shape).

In this case, the intermediate slit 126 extends to be bent. Therefore, it is possible to effectively prevent the second tube 36 from returning from the holding slit 120 to the first insertion hole 100.

### (Fourth Configuration Example)

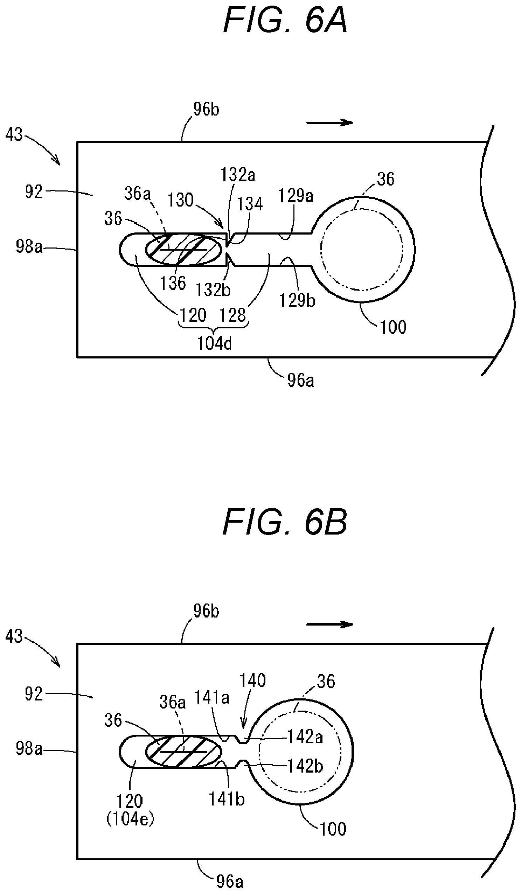

As illustrated in Fig. 6A, the first slit 104d according to the fourth configuration example includes an intermediate slit 128, instead of the intermediate slit 122 of the first slit 104a according to the first configuration example described above. Wall sections 129a and 129b forming the intermediate slit 128 (the first slit 104d) are provided with a return-preventing section 130 that prevents the second tube 36 from returning from the holding slit 120 to the first insertion hole 100.

The return-preventing section 130 has protrusions 132a and 132b which are respectively provided on the wall sections 129a and 129b facing each other and forming the intermediate slit 128. The protrusions 132a and 132b are located on the holding slit 120 side in the intermediate slit 128. The protrusion 132a protrudes from one wall section 129a toward the other wall section 129b. The protrusion 132b protrudes from the other wall section 129b toward the one wall section 129a. The protrusion 132a and the protrusion 132b face each other.

The protrusion 132a is formed in a tapered triangular shape. The protrusion 132a is provided with an inclined portion 134 located on the first insertion hole 100 side and a stopper portion 136 located on the holding slit 120 side.

The inclined portion 134 is inclined toward a side where the first insertion hole 100 is located toward a protruding end of the protrusion 132a. The stopper portion 136 extends substantially perpendicularly to the extending direction of the holding slit 120. The protrusion 132b is configured similarly to the protrusion 132a. Therefore, a configuration of the protrusion 132b will not be described.

In the above first slit 104d, the second tube 36 can move from the first insertion hole 100 to the holding slit 120 over the inclined portions 134 of the protrusions 132a and 132b via the intermediate slit 128. On the other hand, it is difficult for the second tube 36 located in the holding slit 120 to get over the stopper portions 136 of the protrusions 132a and 132b.

In this case, the wall sections 129a and 129b forming the first slit 104d are provided with the return-preventing section 130 that prevents the second tube 36 from returning from the first slit 104d to the first insertion hole 100. Therefore, it is possible to more effectively prevent the second tube 36 from returning from the first slit 104d (holding slit 120) to the first insertion hole 100.

The stopper portion 136 may be inclined to the side opposite to the first insertion hole 100 toward the protruding end of the protrusion 132a. Shapes of the protrusions 132a and 132b are not limited to the tapered triangular shape. The protrusions 132a and 132b may be formed in a semicircular shape. The protrusions 132a and 132b may be located on the first insertion hole 100 side of the intermediate slit 128. The return-preventing section 130 may have only one of the protrusion 132a and the protrusion 132b and omit the other.

### (Fifth Configuration Example)

As illustrated in Fig. 6B, the first slit 104e according to the fifth configuration example includes the holding slit 120 of the first slit 104a according to the first configuration example described above. Note that the first slit 104e does not include the intermediate slit 122 described above.

The wall sections 141a and 141b forming the first slit 104e are provided with a return-preventing section 140 that prevents the second tube 36 from returning from the first slits 104e to the first insertion holes 100. The return-preventing section 140 has protrusions 142a and 142b which are respectively provided on the wall sections 141a and 141b facing each other and forming the first slit 104e.

The protrusions 142a and 142b are located at an end of the first slit 104e on the first insertion hole 100 side. The protrusion 142a protrudes from one wall section 141a toward the other wall section 141b. The protrusion 142b protrudes from the other wall section 141b toward the one wall section 141a. The protrusion 142a and the protrusion 142b face each other. The protrusions 142a and 142b are formed in a semicircular shape.

In this case, the first slit 104e has the same effect as the first slit 104d according to the fourth configuration example described above. In addition, a dimension in the longitudinal direction of the clamp 43 can be made relatively short since the first slit 104e does not have the intermediate slit 122 described above.

### (First Modification)

Next, a clamp 43A according to a first modification will be described. Note that the same components as those of the clamp 43 described above are denoted by the same reference signs in the clamp 43A according to the present modification, and the description thereof will be omitted. The same applies to a clamp 43B according to a second modification and a clamp 43C according to a third modification which will be described later.

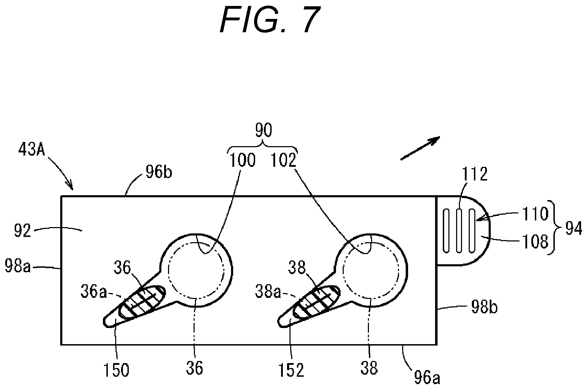

As illustrated in Fig. 7, the clamp 43A according to the first modification includes a first slit 150 and a second slit 152, instead of the first slit 104 and the second slit 106 of the clamp 43 described above. The first slit 150 is different from the first slit 104 only in terms of an extending direction from the first insertion hole 100. That is, the first slit 150 extends so as to be inclined to the first long side 96a side from the first insertion hole 100 toward the first short side 98a.

The second slit 152 is different from the second slit 106 only in terms of an extending direction from the second insertion hole 102. That is, the second slit 152 extends so as to be inclined to the first long side 96a side from the second insertion hole 102 toward the first short side 98a.

According to the clamp 43A of the present modification, a user lifts the operation section 94 obliquely upward in the state of being held by a finger such that the second tube 36 located in the first insertion hole 100 can be easily inserted into the first slit 150 and the third tube 38 located in the second insertion hole 102 can be easily inserted into the second slit 152.

As a shape of the first slit 150 of the clamp 43A according to the present modification, shapes of the first slits 104a to 104e according to the first to fifth configuration examples described above may be adopted.

### (Second Modification)

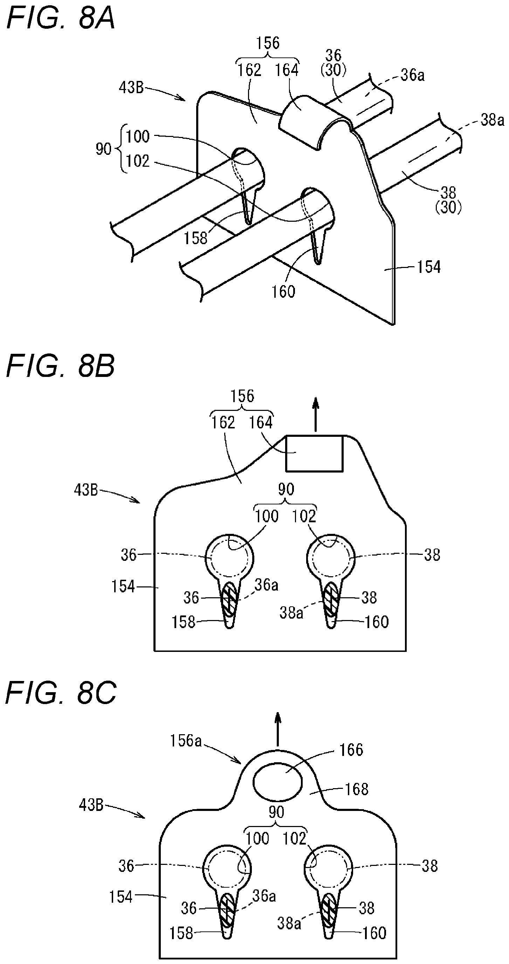

Next, the clamp 43B according to the second modification will be described. As illustrated in Figs. 8A and 8B, the clamp 43B has a rectangular base 154 and an operation section 156 provided on one long side of the base 154. The base 154 is formed with the insertion holes 90 (first insertion hole 100 and second insertion hole 102). A wall section forming the first insertion hole 100 is provided with a first slit 158, and a wall section forming the second insertion hole 102 is provided with a second slit 160.

Each of the first slit 158 and the second slit 160 extends along a direction (lateral direction of the base 154) orthogonal to an array direction of the first insertion hole 100 and the second insertion hole 102. Specifically, the first slit 158 extends from the first insertion hole 100 toward the other long side (side opposite to the operation section 156). A shape of the first slit 158 is similar to the shape of the first slit 104 described above. The second slit 160 extends from the second insertion hole 102 toward the other long side (side opposite to the operation section 156). A shape of the second slit 160 is the same as the shape of the second slit 106 described above.

The operation section 156 includes a flat plate portion 162 (support section) extending from one long side of the base 154 in a direction opposite to the base 154, and a hook portion 164 provided at an extending end of the flat plate portion 162. The hook portion 164 is configured to allow a user to hook a finger thereon, and is curved in an arc shape from the extending end of the flat plate portion 162 to one surface side of the base 154. The hook portion 164 is located closer to the second insertion hole 102 than the center of the base 154 in the longitudinal direction. However, a position of the hook portion 164 in the longitudinal direction of the base 154 can be arbitrarily set.

In the clamp 43B according to the present modification, each of the first slit 158 and the second slit 160 extends along the direction orthogonal to the array direction of the first insertion hole 100 and the second insertion hole 102.

According to such a configuration, as the base 154 slides along the direction orthogonal to the array direction of the first insertion holes 100 and the second insertion holes 102, the second tube 36 located in the first insertion hole 100 can be easily inserted into the first slit 158, and the third tube 38 located in the second insertion hole 102 can be easily inserted into the second slit 160.

The operation section 156 includes the hook portion 164 for hooking the finger. According to such a configuration, a sliding operation of the base 154 can be easily performed.

As illustrated in Fig. 8C, the clamp 43B according to the present modification may include an operation section 156a instead of the operation section 156. The operation section 156a includes a flat plate portion 168 formed with a hole 166 into which the user's finger can be inserted. The hole 166 is located substantially at the center of the base 154 in the longitudinal direction. However, a position of the hole 166 with respect to the longitudinal direction of the base 154 can be arbitrarily set. In such a case where the hole 166 is provided in the flat plate portion 168, the same effect as that of the hook portion 164 is obtained.

As a shape of the first slit 158 of the clamp 43B according to the present modification, the shapes of the first slits 104a to 104e according to the first to fifth configuration examples described above may be adopted.

### (Third Modification)

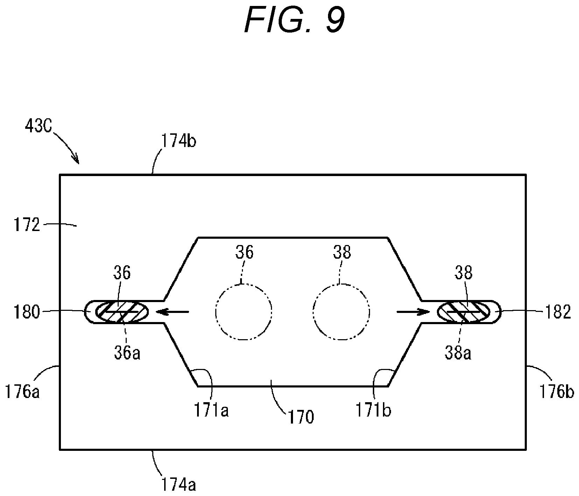

Next, the clamp 43C according to the third modification will be described. As illustrated in Fig. 9, the clamp 43C includes a base 172 formed with one insertion hole 170 through which both the second tube 36 and the third tube 38 are inserted. The base 172 is formed in a rectangular plate shape using a hard resin material. That is, the base 172 includes a first long side 174a and a second long side 174b extending in parallel with each other, and a first short side 176a and a second short side 176b extending in parallel with each other.

The insertion hole 170 is located at the center of the base 172 and extends in the longitudinal direction of the base 172. A first slit 180 for pressing the outer circumferential surface of the second tube 36 inward so as to close the second flow channel 36a of the second tube 36 is provided at one end (end on the first short side 176a side) of the base 172.

The first slit 180 linearly extends from the insertion hole 170 toward the first short side 176a. A slit width of the first slit 180 is smaller than the outer diameter of the second tube 36. The first slit 180 is provided substantially at the center of one end of the base 172 in the lateral direction.

A second slit 182 for pressing the outer circumferential surface of the third tube 38 inward so as to close the third flow channel 38a of the third tube 38 is provided at the other end (end on the second short side 176b side) of the base 172.

The second slit 182 linearly extends from the insertion hole 170 toward the second short side 176b. That is, the first slit 180 and the second slit 182 extend in directions opposite to each other from the insertion hole 170. A slit width of the second slit 182 is smaller than the outer diameter of the third tube 38. The second slit 182 is provided substantially at the center of the other end of the base 172 in the lateral direction.

A wall section forming the insertion hole 170 is provided with a first guide portion 171a that guides the second tube 36 to the first slit 180 and a second guide portion 171b that guides the third tube 38 to the second slit 182. The first guide portion 171a is formed such that one end of the insertion hole 170 (end on the first short side 176a side) becomes narrower toward the first slit 180. The second guide portion 171b is formed such that the other end of the insertion hole 170 (end of the second short side 176b) becomes narrower toward the second slit 182.

In the clamp 43C according to the present modification, the insertion hole 170 is one hole into which both the second tube 36 and the third tube 38 are inserted.

According to such a configuration, the configuration of the clamp 43C can be simplified.

The first slit 180 and the second slit 182 extend in the opposite directions from the insertion hole 170.

According to such a configuration, the second tube 36 and the third tube 38 can be easily inserted into the first slit 180 and the second slit 182, respectively.

As a shape of the first slit 180 of the clamp 43C according to the present modification, the shapes of the first slits 104a to 104e according to the first to fifth configuration examples described above may be adopted.

The present invention is not limited to the above-described embodiments, and various modifications can be made.

## Claims

1. A blood bag system (10) comprising:
a first bag (22) for containing blood;
a second bag (24) for containing a blood component obtained by centrifuging the blood in the first bag (22);
a third bag (26) for containing an additive solution;
**characterized by**
one clamp (43) capable of closing respective flow channels (36a, 38a) of a second tube (36) and a third tube (38),
wherein a first tube (34) connected to the first bag (22) is connected to the second tube (36) connected to the second bag (24) and the third tube (38) connected to the third bag (26) via a branching section (32),
the one clamp (43) includes a plate-shaped base (92; 154),
the base (92; 154) is provided with:
a first insertion hole (100) through which the second tube (36) is inserted;
a second insertion hole (102) through which the third tube (38) is inserted;
a first slit (104; 104a; 104b; 104c; 104d; 104e; 150; 160) which extends from the first insertion hole (100) and which is configured to press an outer circumferential surface of the second tube (36) inward so as to close the flow channel (36a)of the second tube (36); and
a second slit (106; 152; 160) which extends from the second insertion hole (102) and which is configured to press an outer circumferential surface of the third tube (38) inward so as to close the flow channel (38a) of the third tube (38),
the second tube (36) and the third tube (38) are inserted through the base (92),
the base (92) includes the first slit (104; 104a; 104b; 104c; 104d; 104e; 150; 160) and the second slit (106; 152; 160),
the base (92) has a first side (98a) and a second side (98b) located on sides opposite to each other,
the first insertion hole (100) and the second insertion hole (102) are arrayed along a direction from the first side (98a) toward the second side (98b),
the first slit (104; 104a; 104b; 104c; 104d; 104e; 150; 160) and the second slit (106; 152; 160) extend respectively from the first insertion hole (104) and the second insertion hole (106) toward the first side (98a), and
an operation section (94) operable by a finger of a user is provided on the second side (98b).

2. The blood bag system (10) according to claim 1, wherein
the first slit (104; 104a; 104b; 104c; 104d; 104e; 150; 160) includes:
a holding slit (120) that holds the second tube (36); and
an intermediate slit (122, 124, 126, 128) located between the holding slit (120) and the first insertion hole (100), and
a slit width of the intermediate slit (122, 124, 126, 128) is equal to or less than a slit width of the holding slit (120).

3. The blood bag system (10) according to claim 2, wherein
the intermediate slit (126) extends to be bent.

4. The blood bag system (10) according to any one of claims 1 to 3, wherein
a wall section (129a, 129b; 141a, 141b) forming the first slit (104d, 104e) is provided with a return-preventing section (130, 140) that prevents the second tube (36) from returning from the first slit (104d) to the first insertion hole (100).

5. The blood bag system (10) according to any one of claims 1 to 4, wherein
the second slit (106; 152; 160) is formed such that the flow channel (38a) of the third tube (38) is openable as the third tube (38) returns from the second slit (106) to the second insertion hole (102).

6. The blood bag system (10) according to claim 1, wherein
the first slit (104; 104a; 104b; 104c; 104d; 104e; 150; 160) and the second slit (106; 152; 160) are formed to cause the base (92; 154) to slide in one direction with respect to the second tube (36) and the third tube (38) such that the second tube (36) is inserted into the first slit (104; 104a; 104b; 104c; 104d; 104e; 150; 160) and the third tube (38) is inserted into the second slit (106; 152; 160).

7. The blood bag system (10) according to any one of claims 1 to 6, wherein
the operation section (94) includes a flat plate portion (108; 162).

## Patentansprüche

1. Blutbeutelsystem (10) mit:
einem ersten Beutel (22) zum Enthalten von Blut;
einem zweiten Beutel (24) zum Enthalten eines Blutbestandteils, das durch Zentrifugieren des Bluts in dem ersten Beutel (22) erhalten wurde;
einem dritten Beutel (26) zum Enthalten einer Zusatzlösung;
**gekennzeichnet durch**
eine Klemme (43), die in der Lage ist, entsprechende Fließkanäle (36a, 38a) eines zweiten Schlauchs (36) und eines dritten Schlauchs (38) zu schließen,
wobei ein erster Schlauch (34), der mit dem ersten Beutel (22) verbunden ist, mit dem zweiten Schlauch (36) verbunden ist, der mit dem zweiten Beutel (24) verbunden ist, und dem dritten Schlauch (38), der mit dem dritten Beutel (26) verbunden ist, über einen Abzweigabschnitt (32) verbunden ist,
die eine Klemme (43) eine plattenförmige Basis (92, 154) hat,
wobei die Basis (92, 154) bereitgestellt ist mit:
einem ersten Einfügeloch (100), durch das der zweite Schlauch (36) eingefügt ist;
einem zweiten Einfügeloch (102), durch das der dritte Schlauch (38) eingefügt ist;
einem ersten Schlitz (104; 104a; 104b; 104c; 104d; 104e; 150; 160), der sich von dem ersten Einfügeloch (100) erstreckt, und der konfiguriert ist, eine äußere Umfangsoberfläche des zweiten Schlauchs (36) so nach innen zu drücken, dass der Fließkanal (36a) des zweiten Schlauchs (36) geschlossen ist; und
einem zweiten Schlitz (106; 152; 160), der sich von dem zweiten Einfügeloch (102) erstreckt, und der konfiguriert ist, eine äußere Umfangsoberfläche des dritten Schlauchs (38) nach innen zu pressen, um den Fließkanal (38a) des dritten Schlauchs (38) zu schließen,
der zweite Schlauch (36) und der dritte Schlauch (38) durch die Basis (92) eingefügt sind,
die Basis (92) den ersten Schlitz (104; 104a; 104b; 104c; 104d; 104e; 150; 160) und den zweiten Schlitz (106; 152; 160) hat,
die Basis (92) eine erste Seite (98a) und eine zweite Seite (98b) aufweist, die an zueinander gegenüberliegenden Seiten angeordnet sind,
das erste Einfügeloch (100) und das zweite Einfügeloch (102) entlang einer Richtung von der ersten Seite (98a) zu der zweiten Seite (98b) angeordnet sind,
der erste Schlitz (104; 104a; 104b; 104c; 104d; 104e; 150; 160) und der zweite Schlitz (106; 152; 160) sich entsprechend von dem ersten Einfügeloch (104) und dem zweiten Einfügeloch (106) zu der ersten Seite (98a) erstrecken, und
ein durch einen Finger eines Benutzers betätigbarer Betätigungsabschnitt (94) auf der zweiten Seite (98b) bereitgestellt ist.

2. Blutbeutelsystem (10) nach Anspruch 1, wobei
der erste Schlitz (104; 104a; 104b; 104c; 104d; 104e; 150; 160) hat:
einen Halteschlitz (120), der den zweiten Schlauch (36) hält;
einen Zwischenschlitz (122, 124, 16, 128), der zwischen dem Halteschlitz (120) und dem ersten Einfügeloch (100) angeordnet ist, und
eine Schlitzbereite des Zwischenschlitzes (122, 124, 126, 128) gleich groß wie oder kleiner als eine Schlitzbreite des Halteschlitzes (120) ist.

3. Blutbeutelsystem (10) nach Anspruch 2, wobei
der Zwischenschlitz (126) sich erstreckt, um gebogen zu sein.

4. Blutbeutelsystem (10) nach einem der Ansprüche 1 bis 3, wobei
ein Wandabschnitt (129a, 129b; 141a, 141b), der den ersten Schlitz (104d, 104e) ausbildet mit einem Rückkehrverhinderungsabschnitt (130, 140) bereitgestellt ist, der verhindert, dass der zweite Schlauch (36) von dem ersten Schlitz (104d) zu dem ersten Einfügeloch (100) zurückkehrt.

5. Blutbeutelsystem (10) nach einem der Ansprüche 1 bis 4, wobei
der zweite Schlitz (106; 152; 160) derart ausgebildet ist, dass der Fließkanal (38a) des dritten Schlauchs (38) geöffnet werden kann, wenn der dritte Schlauch (38) von dem zweiten Schlitz (106) zu dem zweiten Einfügeloch (102) zurückkehrt.

6. Blutbeutelsystem (10) nach Anspruch 1, wobei
der erste Schlitz (104; 104a; 104b; 104c; 104d; 104e; 150; 160) und der zweite Schlitz (106; 152; 160) ausgebildet sind, um zu verursachen, dass die Basis (92; 154) in eine Richtung mit Bezug auf den zweiten Schlauch (36) und den dritten Schlauch (38) derart gleitet, dass der zweite Schlauch (36) in den ersten Schlitz (1104; 104a; 104b; 104c; 104d; 104e; 150; 160) eigenfügt ist, und der dritte Schlauch (38) in den zweiten Schlitz (106; 152; 106) eingefügt ist.

7. Blutbeutelsystem (10) nach einem der Ansprüche 1 bis 6, wobei
der Betätigungsabschnitt (94) einen flachen Plattenabschnitt (108; 162) hat.

## Revendications

1. Système de poche à sang (10) comprenant :
une première poche (22) destinée à contenir du sang ;
une deuxième poche (24) destinée à contenir un composant sanguin obtenu par centrifugation du sang contenu dans la première poche (22) ;
une troisième poche (26) destinée à contenir une solution d'additif ;
**caractérisé par**
une pince (43) susceptible de fermer des canaux d'écoulement respectifs (36a, 38a) d'un deuxième tube (36) et d'un troisième tube (38),
dans lequel un premier tube (34) relié à la première poche (22) est relié au deuxième tube (36) relié à la deuxième poche (24) et au troisième tube (38) relié à la troisième poche (26) par le biais d'une section de ramification (32),
la pince (43) comprend une base en forme de plaque (92 ; 154),
la base (92 ; 154) est pourvue de :
un premier trou d'insertion (100) à travers lequel est inséré le deuxième tube (36) ;
un second trou d'insertion (102) à travers lequel est inséré le troisième tube (38) ;
une première fente (104 ; 104a ; 104b ; 104c ; 104d ; 104e ; 150 ; 160) qui s'étend depuis le premier trou d'insertion (100) et qui est conçue pour presser une surface circonférentielle externe du deuxième tube (36) vers l'intérieur de manière à fermer le canal d'écoulement (36a) du deuxième tube (36) ; et
une seconde fente (106 ; 152 ; 160) qui s'étend à partir du second trou d'insertion (102) et qui est conçue pour presser une surface circonférentielle externe du troisième tube (38) vers l'intérieur de manière à fermer le canal d'écoulement (38a) du troisième tube (38),
le deuxième tube (36) et le troisième tube (38) sont insérés à travers la base (92),
la base (92) comprend la première fente (104 ; 104a ; 104b ; 104c ; 104d ; 104e ; 150 ; 160) et la seconde fente (106 ; 152 ; 160),
la base (92) comporte un premier côté (98a) et un second côté (98b) situés sur des côtés opposés l'un à l'autre,
le premier trou d'insertion (100) et le second trou d'insertion (102) sont disposés le long d'une direction allant du premier côté (98a) vers le second côté (98b),
la première fente (104 ; 104a ; 104b ; 104c ; 104d ; 104e ; 150 ; 160) et la seconde fente (106 ; 152 ; 160) s'étendent respectivement du premier trou d'insertion (104) et du second trou d'insertion (106) vers le premier côté (98a), et
une section d'actionnement (94) pouvant être actionnée par le doigt d'un utilisateur est prévue sur le second côté (98b).

2. Système de poche à sang (10) selon la revendication 1, dans lequel la première fente (104 ; 104a ; 104b ; 104c ; 104d ; 104e ; 150 ; 160) comprend :
une fente de maintien (120) qui maintient le deuxième tube (36) ; et
une fente intermédiaire (122, 124, 126, 128) située entre la fente de maintien (120) et le premier trou d'insertion (100), et
la largeur de fente de la fente intermédiaire (122, 124, 126, 128) est égale ou inférieure à la largeur de fente de la fente de maintien (120).

3. Système de poche à sang (10) selon la revendication 2, dans lequel la fente intermédiaire (126) s'étend pour être courbée.

4. Système de poche à sang (10) selon l'une quelconque des revendications 1 à 3, dans lequel
une section paroi (129a, 129b ; 141a, 141b) formant la première fente (104d, 104e) est pourvue d'une section empêchant le retour (130, 140) qui empêche le deuxième tube (36) de revenir de la première fente (104d) au premier trou d'insertion (100).

5. Système de poche à sang (10) selon l'une quelconque des revendications 1 à 4, dans lequel
la seconde fente (106 ; 152 ; 160) est formée de telle sorte que le canal d'écoulement (38a) du troisième tube (38) puisse être ouvert à mesure que le troisième tube (38) revient de la seconde fente (106) au second trou d'insertion (102).

6. Système de poche à sang (10) selon la revendication 1, dans lequel
la première fente (104 ; 104a ; 104b ; 104c ; 104d ; 104e ; 150 ; 160) et la seconde fente (106 ; 152 ; 160) sont formées pour amener la base (92 ; 154) à glisser dans une direction par rapport au deuxième tube (36) et au troisième tube (38) de telle sorte que le deuxième tube (36) soit inséré dans la première fente (104 ; 104a ; 104b ; 104c; 104d ; 104e ; 150 ; 160) et que le troisième tube (38) soit inséré dans la seconde fente (106 ; 152 ; 160).

7. Système de poche à sang (10) selon l'une quelconque des revendications 1 à 6, dans lequel
la section d'actionnement (94) comprend une partie de plaque plate (108 ; 162).
